(19) **Europäisches Patentamt / European Patent Office / Office européen des brevets**

(11) **EP 3 913 635 A1**

(12) **EUROPEAN PATENT APPLICATION**
published in accordance with Art. 153(4) EPC

(43) Date of publication:
**24.11.2021 Bulletin 2021/47**

(21) Application number: **19910610.5**

(22) Date of filing: **19.02.2019**

(51) Int Cl.:
**G11C 29/54** *(2006.01)*

(86) International application number:
**PCT/JP2019/006147**

(87) International publication number:
**WO 2020/148918 (23.07.2020 Gazette 2020/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.01.2019 JP 2019006145**

(71) Applicant: **JFE Steel Corporation**
**Tokyo 100-0011 (JP)**

(72) Inventors:
• **TAKAGI Hiroyuki**
  **Tokyo 100-0011 (JP)**
• **YAMAGUCHI Osamu**
  **Tokyo 100-0011 (JP)**
• **NAKATSUJI Kazuhiro**
  **Tokyo 100-0011 (JP)**

(74) Representative: **Grünecker Patent- und Rechtsanwälte**
**PartG mbB**
**Leopoldstraße 4**
**80802 München (DE)**

(54) **METAL MATERIAL DESIGN SUPPORT METHOD AND DESIGN SUPPORT DEVICE**

(57)    A design aid method of aiding in metallic material design by a computer comprises: inputting a desired property value to a database and searching for a chemical composition of elements in metal and a production condition, the database being generated using at least one mathematical model in which input information including a chemical composition of elements in metal and a production condition and output information including a property value of a metallic material are associated with each other, and storing, in association with input data of each mesh obtained by partitioning an input range corresponding to the input information into a plurality of intervals, output data of the mathematical model corresponding to the input data; and presenting the chemical composition of elements in metal and the production condition corresponding to the desired property value that are obtained in the searching.

*FIG. 1*

EP 3 913 635 A1

**Description**

CROSS REFERENCE TO RELATED APPLICATION

This application claims priority to and the benefit of Japanese Patent Application No. 2019-006145 filed on January 17, 2019, the entire disclosure of which is incorporated herein by reference.

TECHNICAL FIELD

The present disclosure relates to a design aid method and a design aid device for a metallic material having a desired property.

BACKGROUND

In conventional metallic material design, to produce a metallic material having a desired property (tensile strength, hardness, toughness, plastic workability, etc.), the chemical composition of elements in metal and the production condition are determined empirically or by trial and error. However, the human load and the temporal load for metallic material design increase as the number of variable items in the chemical composition of elements in metal and the production condition increases.

To reduce the human load and the temporal load, material design using optimized computation and the like by a computer is proposed. For example, JP 4393586 B2 (PTL 1) proposes a method of performing material design using a mathematical model and optimized computation in order to reduce the workload for designing a non-metallic material. JP 5605090 B2 (PTL 2) proposes a material development and analysis device that simulate the property of a substance newly generated by combining a plurality of types of substances, links information of the substances combined and information of the result of simulating the property of the newly generated substance, and extracts specific information according to search criteria input by a user.

CITATION LIST

Patent Literature

> PTL 1: JP 4393586 B2
> PTL 2: JP 5605090 B2

SUMMARY

(Technical Problem)

Metallic material design involves many work processes and device processes as compared with non-metallic material design, and thus requires an enormous amount of computation for management and control relating to the work processes and the device processes. Applying the technique in PTL 1 to metallic material design requires a huge amount of time for optimized computation, which is impractical. Moreover, in metallic material design, there is a possibility that the metallic microstructure of a metallic material changes considerably depending on the production condition, as a result of which the property of the metallic material changes considerably. PTL 1 and PTL 2 fail to take this point into consideration.

It could therefore be helpful to provide a design aid method and a design aid device that can suppress an increase in computation load for metallic material design.

(Solution to Problem)

A design aid method according to one of the disclosed embodiments is a design aid method of aiding in metallic material design by a computer, comprising: inputting a desired property value to a database and searching for a chemical composition of elements in metal and a production condition, the database being generated using at least one mathematical model in which input information including a chemical composition of elements in metal and a production condition and output information including a property value of a metallic material are associated with each other, and storing, in association with input data of each mesh obtained by partitioning an input range corresponding to the input information into a plurality of intervals, output data of the mathematical model corresponding to the input data; and presenting the chemical composition of elements in metal and the production condition corresponding to the desired property value that are obtained in the searching.

A design aid device according to one of the disclosed embodiments is a design aid device that aids in metallic material design, comprising: a search unit configured to input a desired property value to a database and search for a chemical composition of elements in metal and a production condition, the database being generated using at least one mathematical model in which input information including a chemical composition of elements in metal and a production condition and output information including a property value of a metallic material are associated with each other, and storing, in association with input data of each mesh obtained by partitioning an input range corresponding to the input information into a plurality of intervals, output data of the mathematical model corresponding to the input data; and a presentation unit configured to present the chemical composition of elements in metal and the production condition corresponding to the desired property value that are obtained by the search unit.

(Advantageous Effect)

It is thus possible to provide a design aid method and a design aid device that can suppress an increase in computation load for metallic material design.

BRIEF DESCRIPTION OF THE DRAWINGS

In the accompanying drawings:

FIG. 1 is a schematic diagram illustrating a design aid device according to Embodiment 1;
FIG. 2 is a schematic diagram illustrating a steel material production process according to Embodiment 1;
FIG. 3 is a conceptual diagram illustrating mathematical model generation according to Embodiment 1;
FIG. 4 is a conceptual diagram illustrating database generation according to Embodiment 1;
FIG. 5 is a conceptual diagram illustrating a database-based search process according to Embodiment 1;
FIG. 6 is a flowchart illustrating operation of the design aid device according to Embodiment 1;
FIG. 7 is a conceptual diagram illustrating mathematical model generation according to Embodiment 2;
FIG. 8 is a conceptual diagram illustrating a database-based search process according to Embodiment 3;
FIG. 9 is a conceptual diagram illustrating another database-based search process according to Embodiment 3;
FIG. 10 is a conceptual diagram illustrating mathematical model generation according to Embodiment 4;
FIG. 11 is a conceptual diagram illustrating database generation according to Embodiment 5;
FIG. 12 is a scatter diagram of track record values and prediction values for tensile strength; and
FIG. 13 is a scatter diagram of track record values and prediction values for elongation.

DETAILED DESCRIPTION

(Embodiment 1)

Embodiment 1 of the present disclosure will be described below. This embodiment describes an example in which a metallic material to be designed is steel. The metallic material, however, is not limited to steel, and may be any metal.

(Structure of design aid device)

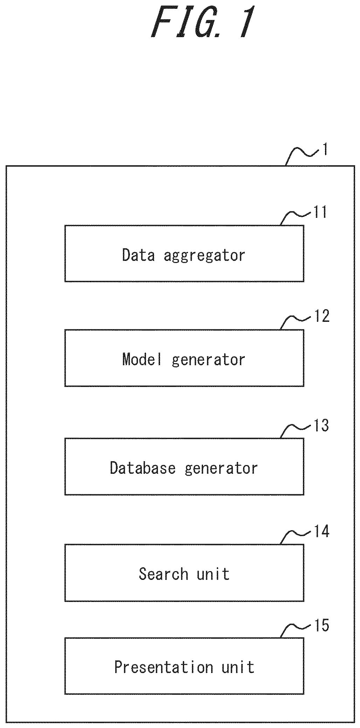

FIG. 1 is a schematic diagram illustrating a design aid device 1 according to Embodiment 1 of the present disclosure. As illustrated in FIG. 1, the design aid device 1 according to Embodiment 1 is a computer including a data aggregator 11, a model generator 12, a database generator 13, a search unit 14, and a presentation unit 15.

The data aggregator 11 aggregates track record data related to steel material production, which is necessary for generating the below-described mathematical model. The data aggregator 11 may include a communication interface for aggregating the track record data. For example, the data aggregator 11 may receive the track record data from a plurality of external devices or the like according to a predetermined communication protocol. The track record data aggregated by the data aggregator 11 includes the chemical composition of elements in steel, the production condition, and the property value of steel material.

The data of the chemical composition of elements in steel aggregated by the data aggregator 11 includes the additive ratios of elements blended as components in steel in a converter or secondary refining. Examples of such elements include C, Si, Mn, P, S, Al, N, Cr, V, Sb, Mo, Cu, Ni, Ti, Nb, B, and Ca.

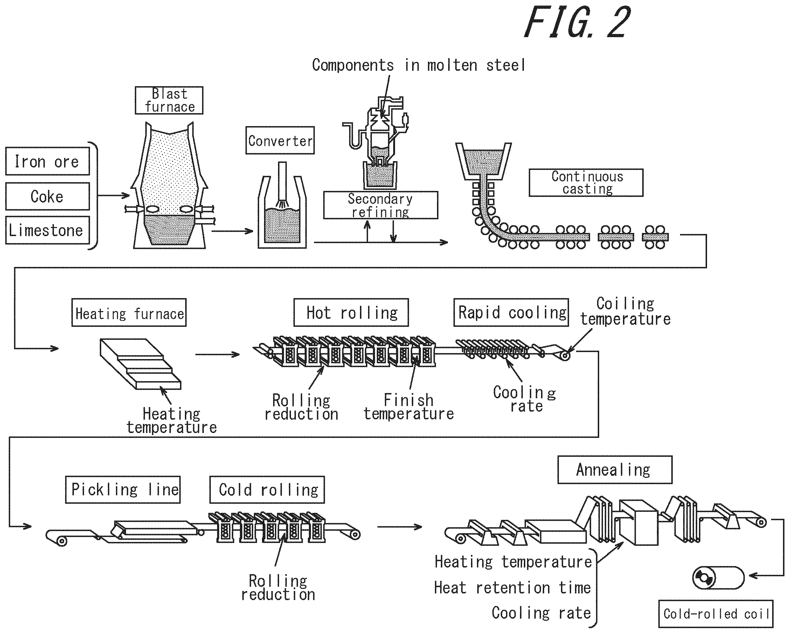

The data of the production condition aggregated by the data aggregator 11 includes various conditions in steps in a steel material production process. FIG. 2 is a schematic diagram illustrating a steel material production process. In the steel production process, first, iron ore as a raw material is charged into a blast furnace together with limestone and coke, to produce pig iron in a molten state. The pig iron produced in the blast furnace undergoes component adjustment for carbon and the like in a converter, and undergoes final component adjustment by secondary refining. The refined steel is cast in a continuous casting machine, to produce an intermediate material called a slab. The slab is then subjected to a plurality of treatment steps such as a heating step in a heating furnace, a hot rolling step, a cooling step, a pickling step, a cold rolling step, an annealing step, and a coating step, to produce a cold-rolled coil as a product. The combination of the plurality of treatment steps differs depending on the product to be produced. Examples of patterns of combining treatment steps include the following:

- Pattern 1: heating step → hot rolling step → cooling step.
- Pattern 2: heating step → hot rolling step → cooling step → pickling step → cold rolling step.
- Pattern 3: heating step → hot rolling step → cooling step → pickling step → cold rolling step → annealing step.
- Pattern 4: heating step → hot rolling step → cooling step → pickling step → cold rolling step → annealing step → coating step → heat treatment step.

Examples of the conditions in the steps, i.e. the production condition, include the following:

- Heating step: heating temperature, heating time.
- Hot rolling step: thickness, sheet width, cumulative rolling reduction, rolling start temperature, rolling finish temperature, coiling temperature, cooling rate.
- Cooling step: cooling start temperature, cooling rate.
- Pickling step: pickling chemical solution concentration, pickling chemical solution temperature, pickling rate.
- Cold rolling step: thickness, sheet width, rolling reduction.
- Annealing step: heating rate, heating temperature, holding time, cooling rate, cooling method.
- Coating step: hot dip coating temperature, coating adjustment gas blowing amount.
- Heat treatment step: heating rate, heating temperature.

The data of the property value of steel material aggregated by the data aggregator 11 includes, for example, tensile strength, yield point, elongation, hardness, impact absorbed energy, r value, n value, hole expansion ratio, and BH amount. For example, the property value can be obtained by conducting a sampling test of evaluating, from part of the produced steel material product, the property of the steel material.

The data aggregator 11 manages the aggregated track record data in association. In other words, for each unit of steel material product produced, the data aggregator 11 links the track record data of the chemical composition of elements in steel, the track record data of the production condition, and the track record data of the property value of steel material in an integrated manner so that these data can be handled collectively.

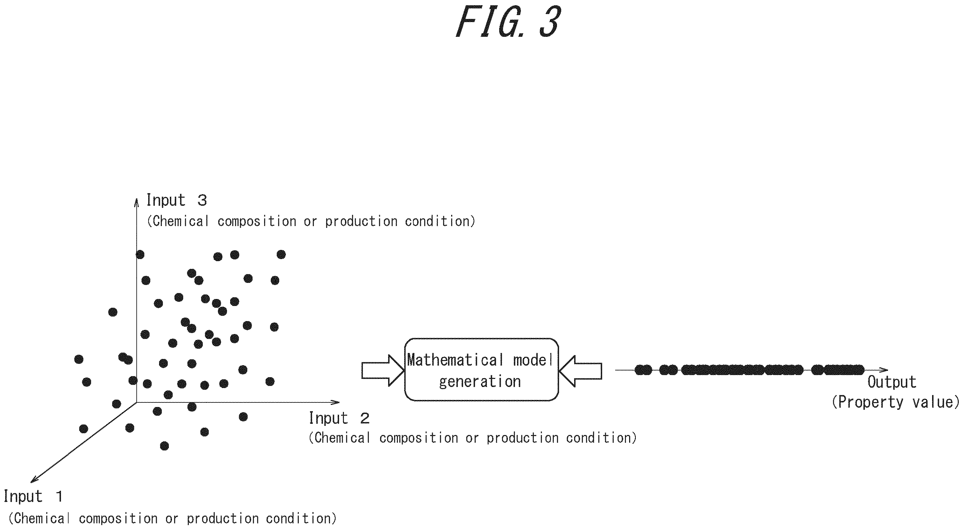

The model generator 12 generates a mathematical model associating input information including the chemical composition of elements in steel and the production condition and output information including the property value of steel material with each other, based on the track record data aggregated by the data aggregator 11. Herein, the term "input information" denotes information of track record values used to generate the mathematical model, and the term "output information" denotes information of track record values used to generate the mathematical model. FIG. 3 is a conceptual diagram illustrating mathematical model generation according to Embodiment 1. As illustrated in FIG. 3, the model generator 12 associates input information and output information with each other to generate a mathematical model. Here, the model generator 12 generates the mathematical model based on the track record data according to any algorithm. As the algorithm for generating the mathematical model, for example, statistical methods and machine learning methods such as local regression, support vector machine, neural network, and random forest can be used. In FIG. 3, three pieces of input information, i.e. input 1 to input 3, and one piece of output information are illustrated for the sake of simplicity. The input 1 to input 3 each correspond to the chemical composition of elements in steel or the production condition from among the track record data aggregated by the data aggregator 11, and the output corresponds to the property value of steel material in the track record data.

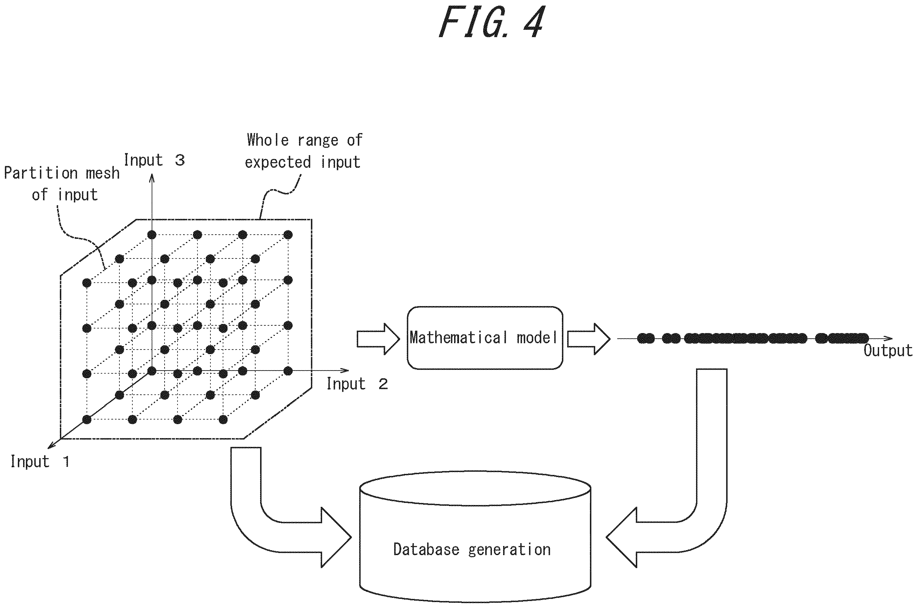

The database generator 13 generates a database using the mathematical model generated by the model generator 12. FIG. 4 is a conceptual diagram illustrating database generation according to Embodiment 1. The database generator 13 partitions the input data range for the input information into a plurality of intervals to define input data meshes, and determines a representative value corresponding to each input data mesh as input data. The representative value may be any value representative of the mesh, such as a median value or an end value, e.g. an upper limit value or a lower limit value, in the mesh. The input data range need not necessarily be the same as the input information that is the track record data. The database generator 13 inputs input data of each mesh defined in this way to the mathematical model generated by the model generator 12, to obtain output data of the mesh. Herein, the output data is the output value of the model corresponding to the input data. The input data of each mesh may be, for example, a median value of the data interval defined as the mesh, as a representative value. The database generator 13 stores, for each mesh, the correspondence between the input data of the mesh and the output data obtained by inputting the input data to the mathematical model, thus generating a database. That is, the database generator 13 generates the database in which the output data of each mesh obtained by partitioning the input data range into the plurality of intervals is stored.

As the input data range in which the input data meshes are defined, chemical compositions of elements in steel and production conditions that are expected as steel material are taken to be the whole input range. That is, the input data range is limited to a predetermined range based on a predetermined condition such as metallurgical knowledge or evaluation function. Table 1 shows an example of limitations regarding the input data range.

[Table 1]

| Production step | Design conditions | Lower limit value | Upper limit value |
|---|---|---|---|
| Converter and secondary refining | C (%) | **** | **** |
| | Si (%) | **** | **** |
| | Mn (%) | **** | **** |
| | P (%) | **** | **** |
| | S (%) | **** | **** |
| | Cu (%) | **** | **** |
| | Ni (%) | **** | **** |
| | Cr (%) | **** | **** |
| | Sb (%) | **** | **** |
| | Sn (%) | **** | **** |
| Heating step | Heating temperature (°C) | **** | **** |
| Hot rolling step | Rolling reduction | **** | **** |
| | Finish temperature (°C) | **** | **** |
| | Coiling temperature (°C) | **** | **** |
| | Cooling rate (°C/s) | **** | **** |
| Cold rolling step | Rolling reduction | **** | **** |
| Annealing step | Heating temperature (°C) | **** | **** |
| | Heat retention time (s) | **** | **** |
| | Cooling rate (°C/s) | **** | **** |

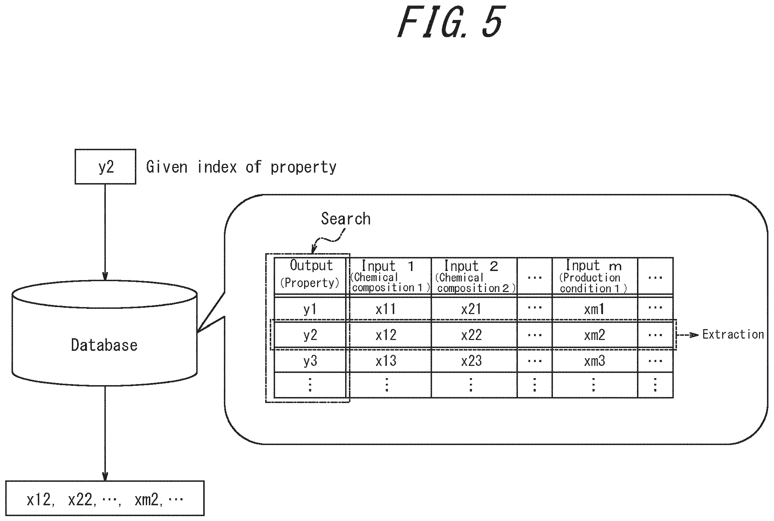

The search unit 14 searches the database for input data corresponding to a given index, on the condition that the given index and output data match. FIG. 5 is a conceptual diagram illustrating a database-based search process according to Embodiment 1. FIG. 5 illustrates an example in which one piece of output data is associated with m or more pieces of input data. The search unit 14 searches the database using a designated desired property value as an index, and obtains, as a search result, a chemical composition of elements in steel and a production condition in a plurality of steps corresponding to the desired property value. In FIG. 5, the database is searched using a desired property value "y2" as an index, and a search result "x12, x22, ... xm2, ..." is obtained. Herein, the "chemical composition of elements in steel and production condition in a plurality of steps corresponding to the desired property value" include not only a chemical composition of elements in steel and a production condition in a plurality of steps that achieve a property value matching the desired property value designated as the index, but also a chemical composition of elements in steel and a production condition in a plurality of steps that achieve a property value similar to the desired property value designated as the index. Herein, similar property values are property values having a small absolute difference therebetween. The range of similarity can be set for each type of property value. Thus, in the case where there is data in a range similar to the value given as the desired property value, the search result is output with the data being regarded as matching the desired property value.

In FIG. 5, one search result is obtained as a result of the search using "y2" as the index. However, the present disclosure is not limited to such. For example, in the case where one or more other pieces of input data also correspond to the property value "y2", a plurality of search results may be extracted. In this way, a plurality of patterns of a chemical composition of elements in steel and a production condition in a plurality of steps that achieve the desired property can be obtained. This enhances the possibility of efficient steel material design.

The presentation unit 15 presents, to a user, the search result by the search unit 14, i.e. the chemical composition of elements in steel and the production condition corresponding to the desired property value. The user can efficiently design a steel material using, as a target value or a reference value in steel material production, the chemical composition of elements in steel and the production condition in a plurality of steps presented by the presentation unit 15.

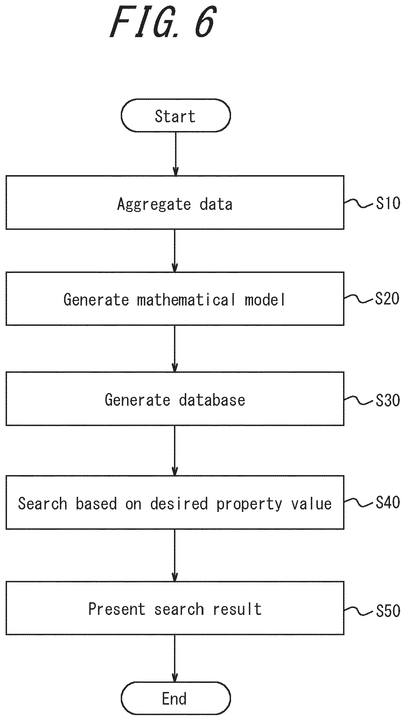

Operation of the design aid device 1 according to Embodiment 1 will be described below, with reference to a flowchart in FIG. 6.

First, the data aggregator 11 aggregates track record data necessary to generate a mathematical model (step S10). The track record data aggregated by the data aggregator 11 includes data relating to a produced steel material such as the chemical composition of elements in steel, the production condition, and the property value of steel material.

Next, the model generator 12 generates a mathematical model associating input information including the chemical composition of elements in steel and the production condition and output information including the property value of steel material with each other, based on the track record data aggregated by the data aggregator 11 (step S20).

Next, the database generator 13 generates a database for aiding in steel material design, using the mathematical model generated by the model generator 12 (step S30). Specifically, the database generator 13 generates a database in which output data corresponding to input data of each mesh obtained by partitioning an input data range into a plurality of intervals is stored in association with the input data.

Next, the search unit 14 searches the database for a chemical composition of elements in steel and a production condition corresponding to a desired property value (step S40).

Next, the presentation unit 15 presents the chemical composition of elements in steel and the production condition corresponding to the desired property value, which are obtained as a result of the search by the search unit 14 (step S50).

Thus, the design aid device 1 according to Embodiment 1 uses a database storing output data of each mesh obtained by partitioning an input data range into a plurality of intervals, instead of performing optimized computation. The design aid device 1 according to Embodiment 1 then searches the database for a chemical composition of elements in metal and a production condition corresponding to a desired property value, and presents the chemical composition of elements in metal and the production condition corresponding to the desired property value. The design aid device 1 according to Embodiment 1 can aid in design without performing optimized computation, and therefore can suppress an increase in computation load for steel material design.

(Embodiment 2)

Embodiment 2 of the present disclosure will be described below. The same components as those in Embodiment 1 are given the same reference signs, and their description is omitted. A design aid device 1 according to Embodiment 2 differs from the structure according to Embodiment 1 in the contents of track record data aggregated by the data aggregator 11.

The track record data aggregated by the data aggregator 11 in the design aid device 1 according to Embodiment 2 includes an index indicating metallic microstructure state, in addition to a chemical composition of elements in steel, a production condition, and a property value of steel material. Examples of the index indicating metallic microstructure state include the grain size and microstructure proportion of ferrite, the microstructure proportion of cementite, the microstructure proportion of pearlite, the microstructure proportion of bainite, and the microstructure proportion of martensite. Any method may be used to aggregate the index indicating metallic microstructure state. For example, the data aggregator 11 may obtain the index indicating metallic microstructure state by conducting a sampling test of evaluating, from part of the produced steel material product, the index indicating metallic microstructure state. The data aggregator 11 may then associate the data of the index obtained in this way with the production data of the steel material product and the property of the steel material. Alternatively, the data aggregator 11 may obtain the index indicating metallic microstructure state by a measurement device capable of evaluating the index indicating metallic microstructure state, during production. The data aggregator 11 may then associate the data of the index obtained in this way with the production data of the product and the property of the steel material. Alternatively, the data aggregator 11 may obtain the index indicating metallic microstructure state by simulation with which the index indicating metallic microstructure state can be evaluated, during production. The data aggregator 11 may then associate the data of the index obtained in this way with the production data of the product and the property of the steel material.

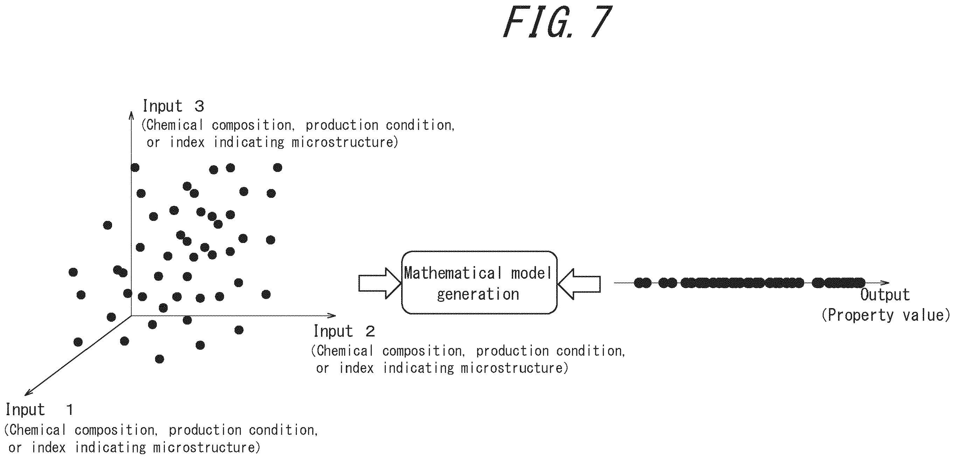

The model generator 12 in the design aid device 1 according to Embodiment 2 generates a mathematical model associating input information including the chemical composition of elements in steel, the production condition, and the index indicating metallic microstructure state and output information including the property value of steel material with each other. FIG. 7 is a conceptual diagram illustrating mathematical model generation according to Embodiment 2. In FIG. 7, three pieces of input information, i.e. input 1 to input 3, are illustrated for the sake of simplicity. The input 1 to input 3 each correspond to the chemical composition of elements in steel, the production condition, or the index indicating metallic microstructure state. As illustrated in FIG. 7, the model generator 12 associates input information and output information to generate a mathematical model. The database generation by the database generator 13 is the same as that in Embodiment 1, and accordingly its description is omitted. The search process by the search unit 14 involves searching the database using a designated desired property value as an index and obtaining, as a search result, an index indicating metallic microstructure state in addition to a chemical composition of elements in steel and a production condition corresponding to the desired property value. The information presentation process by the presentation unit 15 is the same as that in Embodiment 1, and accordingly its description is omitted.

The design aid device 1 according to Embodiment 2 uses the data of metallic microstructure state which is a direct factor for achieving the property of steel material, and thus can improve the accuracy of the mathematical model generated. Moreover, the design aid device 1 according to Embodiment 2 obtains, as the search result, the index indicating metallic microstructure state in addition to the chemical composition of elements in steel and the production condition that achieve the desired property value, and thus can improve the accuracy of steel material design based on the information of the metallic microstructure state. The design aid device 1 according to Embodiment 2 can therefore accurately determine the chemical composition of elements in metal and the production condition with which the desired property value of steel material can be achieved, and perform high-accuracy design.

(Embodiment 3)

Embodiment 3 of the present disclosure will be described below. The same components as those in Embodiment 1 are given the same reference signs, and their description is omitted. A design aid device 1 according to Embodiment 3 differs from the structure according to Embodiment 1 in that the model generator 12 generates a mathematical model for each property value.

Examples of the property of metallic material include tensile strength, yield point, elongation, hardness, impact absorbed energy, r value, n value, hole expansion ratio, and BH amount, as described in Embodiment 1. The model generator 12 in the design aid device 1 according to Embodiment 3 generates a mathematical model for each of such a plurality of types of properties separately. In other words, the model generator 12 in the design aid device 1 according to Embodiment 3 generates a plurality of mathematical models.

The database generator 13 generates a database using the plurality of mathematical models generated by the model generator 12. Specifically, the database generator 13 sets chemical compositions of elements in steel and production conditions that are expected as steel material as the whole input range, and partitions the input range into a plurality of intervals to define input data meshes. The input data range of data input to the database need not necessarily match the range of input information. It is assumed here that input data is a representative value of each data mesh (as in Embodiment 1). The database generator 13 inputs input data of each defined mesh to each of the plurality of mathematical models generated by the model generator 12, to obtain output data of the mesh. The database generator 13 stores, for each mesh, the correspondence between the input data of the mesh and the output data obtained by inputting the input data to each of the plurality of mathematical models, thus generating a database. In other words, the database generator 13 generates a database in which the output data of each mesh obtained by partitioning the input data range into the plurality of intervals is stored.

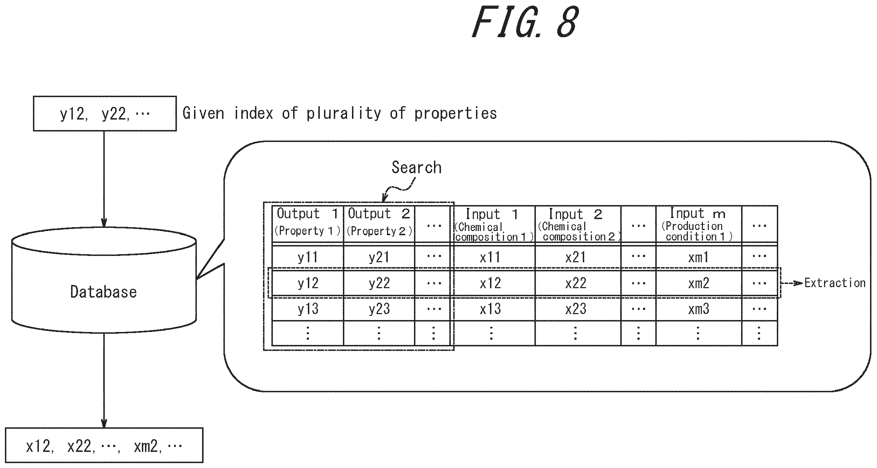

The search process by the search unit 14 is the same as that in Embodiment 1, except that the index used in the search can be designated from a plurality of types of properties. FIG. 8 is a conceptual diagram illustrating a database-based search process according to Embodiment 3. In FIG. 8, the database is searched using a desired property value "y12, y22, ..." as an index, and a search result "x12, x22, ... xm2, ..." is obtained. The presentation unit 15 presents the search result to the user. In other words, the presentation unit 15 presents the chemical composition of elements in steel and the production condition corresponding to the plurality of desired property values, which are obtained as a result of the search by the search unit 14. The user can efficiently design a steel material using, as a target value or a reference value in steel material production, the chemical composition of elements in steel and the production condition in a plurality of steps presented by the presentation unit 15.

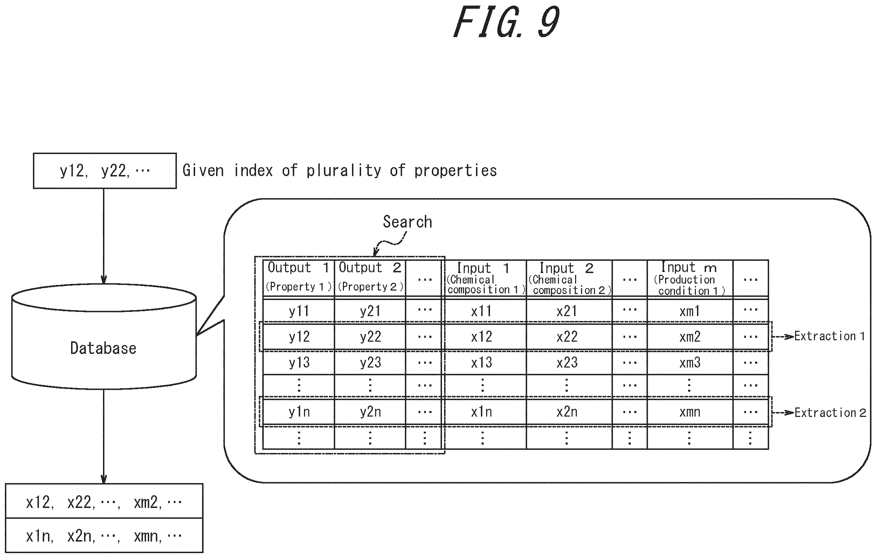

FIG. 9 is a conceptual diagram illustrating another database-based search process according to Embodiment 3. In FIG. 9, search is performed with a desired property value "y12, y22, .." as an index, as in FIG. 8. Suppose there is no search result that matches the index. In this case, the search unit 14 outputs at least one search result having output data in a range similar to the index. For example, in the case where there is output data matching part of the plurality of desired property values as the index, the search unit 14 presents data having output data that matches the part and is similar to any other non-matched property, as a candidate. Here, the degree of similarity may be determined, for example, based on the distance of a vector formed by the non-matched property value. Alternatively, for example, the search unit 14 outputs data having output data most similar to any one of the plurality of desired property values as the index, as a search result. In FIG. 9, two search result candidates "x12, x22, ..., xm2, ..." and "xln, x2n, ..., xmn, ..." are output. The presentation unit 15 may present the two search results to the user. Although the number of search result candidates is two in this example, the number of search results presented is not limited to such, and may be three or more. Regarding the range of similarity, the value between the elements of the foregoing vector may be normalized, and the distance of the normalized vector may be set to a predetermined distance. The range of similarity may be determined for each element.

The design aid device 1 according to Embodiment 3 can easily determine a complex input-output relationship of a chemical composition of elements in steel and a production condition in a plurality of steps that achieve a plurality of properties of steel material, so that steel material design can be performed efficiently.

(Embodiment 4)

Embodiment 4 of the present disclosure will be described below. The same components as those in Embodiment 1 are given the same reference signs, and their description is omitted. A design aid device 1 according to Embodiment 4 differs from the structure according to Embodiment 1 in the contents of track record data aggregated by the data aggregator 11 and the structure of the mathematical model generated by the model generator 12.

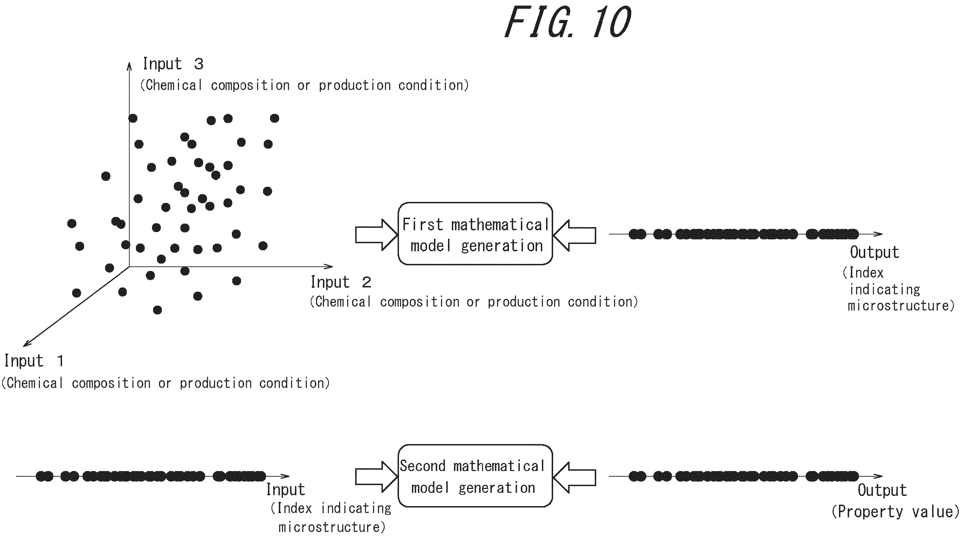

The track record data aggregated by the data aggregator 11 in the design aid device 1 according to Embodiment 4 includes an index indicating metallic microstructure state, in addition to a chemical composition of elements in steel, a production condition, and a property value of steel material. The model generator 12 generates a first mathematical model associating input information including the chemical composition of elements in steel and the production condition and intermediate output information including the index indicating metallic microstructure state with each other, and a second mathematical model associating the intermediate output information and output information including the property of metallic material with each other. FIG. 10 is a conceptual diagram illustrating mathematical model generation according to Embodiment 4. In Embodiment 4, using the index indicating metallic microstructure state as the output information (intermediate output information) of the first mathematical model, the input information and the output information are associated with each other via the intermediate output information in the first and second mathematical models, as illustrated in FIG. 10.

The database generator 13 generates a database using the plurality of mathematical models generated by the model generator 12, i.e. the first and second mathematical models. Specifically, the database generator 13 sets chemical compositions of elements in steel and production conditions that are expected as steel material as the whole input range, and partitions the input range into a plurality of intervals to define input data meshes. The input data range of data input to the database need not necessarily match the range of input information. It is assumed here that input data is a representative value of each data mesh (as in Embodiment 1). The database generator 13 inputs input data of each defined mesh to the first mathematical model, to obtain intermediate output data of the mesh. The database generator 13 then inputs the intermediate output data to the second mathematical model, to obtain output data of the mesh. The database generator 13 stores, for each mesh, the correspondence between the input data of the mesh and the output data obtained by inputting the input data to each of the plurality of mathematical models, thus generating a database. In other words, the database generator 13 generates a database in which the output data of each mesh obtained by partitioning the input data range into the plurality of intervals is stored. In the search process by the search unit 14, for example, search is performed for a range of an index indicating metallic microstructure state as intermediate output that is limited to a predetermined range using the desired property value as an index, and then search is performed for a chemical composition of elements in steel and a production condition using, as an index, the index indicating metallic microstructure obtained as a result of the search in the limited range. Alternatively, in the search process by the search unit 14, search is performed for a range of an index indicating metallic microstructure state as intermediate output using a predetermined range of the desired property value as an index, and then search is performed for a plurality of candidates for a chemical composition of elements in steel and a production condition using, as an index, the range of the index indicating metallic microstructure state obtained as a result of the search. In other words, the search unit 14 searches the database for an index indicating metallic microstructure state corresponding to the desired property value and a chemical composition of elements in metal and a production condition corresponding to the index indicating metallic microstructure state. The information presentation process by the presentation unit 15 is the same as that in Embodiment 1, and accordingly its description is omitted.

The design aid device 1 according to Embodiment 4 uses, as intermediate output, the information of metallic microstructure state which is a direct factor for achieving the property of steel material, and thus can improve the accuracy of the mathematical model generated. The design aid device 1 according to Embodiment 4 can therefore accurately determine the chemical composition of elements in metal and the production condition with which the desired property value of steel material can be achieved, and perform high-accuracy design.

(Embodiment 5)

Embodiment 5 of the present disclosure will be described below. The same components as those in Embodiment 1 are given the same reference signs, and their description is omitted. A design aid device 1 according to Embodiment 5 differs from the structure according to Embodiment 1 in the input data meshes of the database generated by the database generator 13.

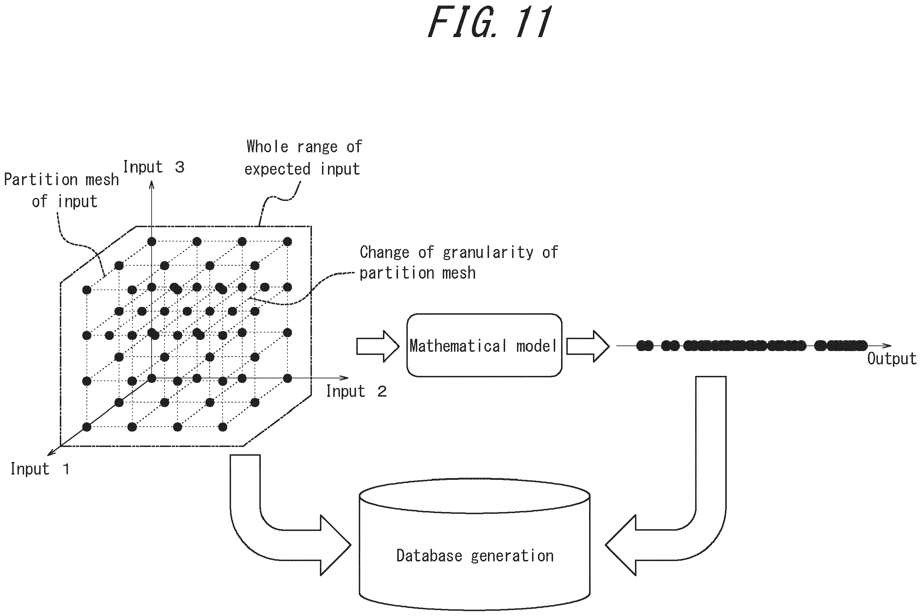

FIG. 11 is a conceptual diagram illustrating database generation according to Embodiment 5. The database generator 13 sets chemical compositions of elements in steel and production conditions that are expected as steel material as the whole input range, and partitions the input range into a plurality of intervals to define input data meshes. Here, the database generator 13 varies the granularity (interval width) of the interval of each mesh depending on the type of input data. For example, the database generator 13 may change the interval width for each item beforehand so that the interval width is fine for an important item and coarse for an unimportant item, based on metallurgical knowledge. The database generator 13 may change the interval width based on the data density of each item. It is metallurgically known that the tensile strength which is one of the properties of steel material is particularly sensitive to the chemical composition of carbon in steel. Hence, the database generator 13 may set a fine mesh interval width in the design of the chemical composition of carbon. Alternatively, the database generator 13 may set the interval width of the interval of each mesh so that the amount of change in output will be constant. In other words, the database generator 13 may set the interval width of each mesh so that the difference in output between adjacent meshes will be constant.

Thus, the design aid device 1 according to Embodiment 5 stores only the data of the minimum number of meshes as the database, so that the computation load and the computation time in model generation and the search load and the search time in design can be reduced. That is, a huge computation load and search load that can be caused in the case where the mesh interval width is relatively fine (for example, 0.001) for every item can be avoided. A decrease in accuracy of designing a steel material that achieves a desired property value, which can be caused in the case where the mesh interval width is relatively coarse (for example, 0.01) for every item, can be avoided, too. Hence, the steel material that achieves the desired property value can be designed efficiently and accurately with a minimum load.

EXAMPLES

An example of design of a steel material for a cold-rolled steel sheet for a vehicle will be described below. In this example, tensile strength and elongation are selected as properties of the steel material, and search is performed for a design condition that achieves desired property values.

Table 2 shows examples of chemical compositions of elements in steel influencing the properties. Table 3 shows examples of production conditions influencing the properties. Table 4 shows property types and property values. The track record data items in Tables 2 to 4 are aggregated and machine learning is performed using these data to construct a mathematical model having a chemical composition and a production condition as input and a property as output.

[Table 2]

(unit: mass%)

| Steel sample ID | C | Si | Mn | P | S | Al | N | Cr | V | Sb | Mo | Cu | Ni | Ti | Nb | B | Ca |
|---|---|---|---|---|---|---|---|---|---|---|---|---|---|---|---|---|---|
| A | 0.124 | 0.66 | 2.55 | 0.008 | 0.001 | 0.037 | 0.0034 | 0 | 0 | 0.011 | 0 | 0 | 0 | 0.015 | 0.038 | 0.0016 | 0.0002 |
| B | 0.105 | 0.53 | 2.79 | 0.01 | 0.0008 | 0.035 | 0.004 | 0 | 0 | 0.01 | 0 | 0 | 0 | 0.014 | 0.042 | 0.0015 | 0.0001 |
| C | 0.131 | 0.56 | 2.57 | 0.009 | 0.0011 | 0.042 | 0.0036 | 0.05 | 0 | 0.009 | 0 | 0 | 0 | 0.017 | 0.034 | 0.0017 | 0.0001 |

[Table 3]

| Steel sheet No. | Steel sample ID | Hot rolling condition | | | Continuous annealing condition | | | | | | | | | | |
|---|---|---|---|---|---|---|---|---|---|---|---|---|---|---|---|
| | | Heating temperature (°C) | Finish rolling temperature (°C) | Coiling temperature (°C) | Thickness (mm) | Average heating rate in temperature range of 570 ° C or more (°C/s) | Heating temperature (°C) | Soaking temperature (°C) | Holding time in temperature range of Ac3 or more (s) | Average cooling rate in temperature range of 620 ° C to 740 °C (°C/s) | Cooling stop temperature (°C) | Holding time in temperature range of 620 ° C to 740 °C (s) | Average cooling rate in temperature range of 400 ° C or less (°C/s) | Cooling stop temperature (°C) | Holding time in temperature range of 150 °C or more and 400 ° C on less (s) |
| 1 | A | 1240 | 880 | 560 | 1.4 | 4 | 620 | 860 | 140 | 1.8 | 660 | 18 | 37 | 280 | 430 |
| 2 | B | 1240 | 880 | 560 | 1.4 | 4 | 630 | 860 | 110 | 3.4 | 680 | 37 | 18 | 310 | 510 |
| 3 | C | 1240 | 880 | 560 | 1.4 | 4 | 620 | 850 | 120 | 1.5 | 680 | 22 | 22 | 260 | 470 |

[Table 4]

| Steel sheet No. | Steel sample ID | Tensile strength (MPa) | Elongation (%) |
|---|---|---|---|
| 1 | A | 1283 | 11.2 |
| 2 | B | 1205 | 12.2 |
| 3 | C | 1247 | 9.8 |

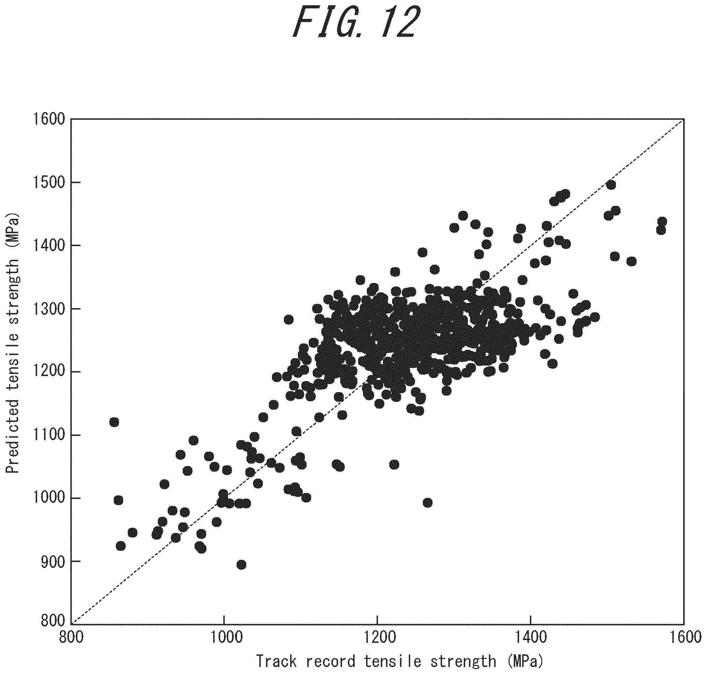

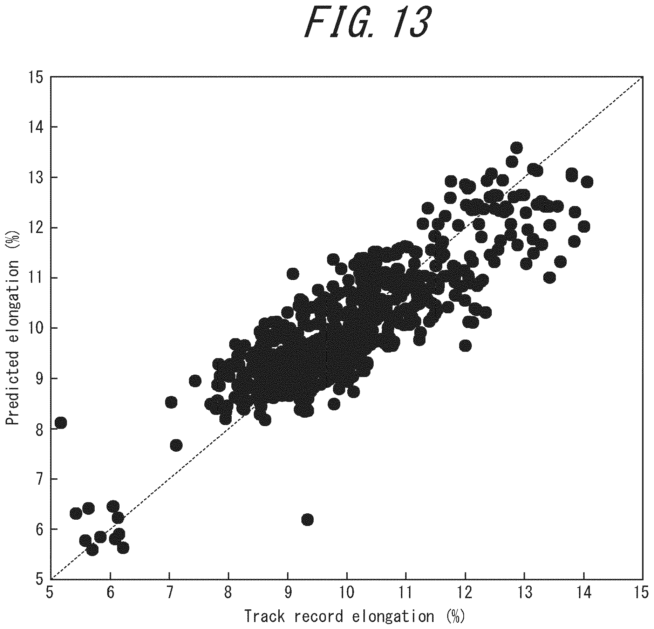

In this example, using 500 entries of learning data, respective mathematical models for predicting tensile strength and elongation as properties were generated using a machine learning method called random forest. FIGS. 12 and 13 are each a scatter diagram of track record values and prediction values. In the scatter diagram in FIG. 12, the horizontal axis represents the track record value of tensile strength, and the vertical axis represents the prediction value of tensile strength. In the scatter diagram in FIG. 13, the horizontal axis represents the track record value of elongation, and the vertical axis represents the prediction value of elongation. The number of regression tree used in a random forest was 50 in each mathematical model. The prediction accuracy of the model for tensile strength prediction evaluated by leave-one-out cross-validation was 81.5 in RMSE (root mean square error). The prediction accuracy of the model for elongation prediction was 0.729 in RMSE. Herein, RMSE is an index of a prediction error calculated as follows:

[Math. 1]

$$RMSE = \sqrt{\frac{1}{N}\sum_{i=1}^{N}(y_i - \hat{y_i})^2}$$

where N is the total number of prediction targets, yi is a track record value, and y^i is a prediction value.

Following this, input data of each defined mesh was input to the generated mathematical model, to obtain output data of the mesh. Here, the mesh interval width of the chemical composition (unit: mass%) of C, P, Al, Sb, Ti, and Nb in the input data was set to 0.001 %, the mesh interval width of the chemical composition (unit: mass%) of S, N, B, and Ca was set to 0.0001 %, and the mesh interval width of the chemical composition (unit: mass%) of the other elements was set to 0.01 %. A database generated by storing the correspondence between input data and output data for each mesh was searched using desired property values of the properties of steel material as an index. For example, the desired property values set as shown in Table 5 were read as the index.

[Table 5]

| Property | Desired property value |
|---|---|
| Tensile strength (MPa) | 1200 |
| Elongation (%) | 12.0 |

Thus, the learned plurality of mathematical models and the desired property values of the properties of steel material used in design condition search with the mesh interval width were obtained, enabling obtainment of a chemical composition of elements in steel and a production condition in a plurality of steps that achieve the desired property values of steel material.

Input (chemical composition of elements in steel and production condition in a plurality of steps) obtained as a result of search is shown in Table 6. A steel product produced under this design condition had a tensile strength of 1200 MPa and an elongation of 12.0 %. A steel material achieving the desired property values was thus designed successfully.

[Table 6]

| Production step | Item | Value |
|---|---|---|
| Converter and secondary refining | C (%) | 0.104 |
| | Si (%) | 0.54 |
| | Mn (%) | 2.79 |
| | P (%) | 0.01 |
| | S (%) | 0.0007 |
| | Al (%) | 0.035 |
| | N (%) | 0.0041 |
| | Cr (%) | 0 |
| | V (%) | 0 |
| | Sb (%) | 0.01 |
| | Mo (%) | 0 |
| | Cu (%) | 0 |
| | Ni (%) | 0 |
| | Ti (%) | 0.015 |
| | Nb (%) | 0.04 |
| | B (%) | 0.0016 |
| | Ca (%) | 10.0001 |
| Heating furnace and hot rolling | Heating temperature (°C) | 1235 |
| | Finish rolling temperature (°C) | 879 |
| | Coiling temperature (°C) | 561 |
| Annealing | Thickness (mm) | 1.4 |
| | Average heating rate in temperature range of 570 °C or more (°C/s) | 4 |
| | Heating temperature (°C) | 632 |
| | Soaking temperature (°C) | 861 |
| | Heat retention time in temperature range of Ac3 or more (s) | 109 |
| | Average cooling rate in temperature range of 620 °C to 740 °C (°C/s) | 3.3 |
| | Cooling stop temperature (°C) | 684 |
| | Heat retention time in temperature range of 620 °C to 740 °C (s) | 37 |
| | Average cooling rate in temperature range of 400 °C or less (°C/s) | 18 |
| | Cooling stop temperature (°C) | 308 |
| | Heat retention time in temperature range of 150 °C or more and 400 °C or less (s) | 518 |

As Comparative Example 1, a search result in the case of setting the mesh interval width of the chemical composition of every element in the input data to 0.01 % is shown in Table 7. A steel product produced under this design condition had a tensile strength of 1240 MPa and an elongation of 11.5 %. This demonstrates that Example 1 is more preferable in designing a steel material that achieves the desired property values.

[Table 7]

| Production step | Item | Lower limit |
|---|---|---|
| Converter and secondary refining | C (%) | 0.11 |
| | Si (%) | 0.54 |
| | Mn (%) | 2.81 |
| | P (%) | 0.01 |
| | S (%) | 0 |
| | Al (%) | 0.04 |
| | N (%) | 0 |
| | Cr (%) | 0 |
| | V (%) | 0 |
| | Sb (%) | 0.01 |
| | Mo (%) | 0 |
| | Cu (%) | 0 |
| | Ni (%) | 0 |
| | Ti (%) | 0.01 |
| | Ni (%) | 0.04 |
| | B (%) | 0 |
| | Ca (%) | 10 |
| Heating furnace and hot rolling | Heating temperature (°C) | 1235 |
| | Finish rolling temperature (°C) | 874 |
| | Coiling temperature (°C) | 555 |
| Annealing | Thickness (mm) | 1.4 |
| | Average heating rate in temperature range of 570 °C or more (°C/s) | 4 |
| | Heating temperature (°C) | 626 |
| | Soaking temperature (°C) | 858 |
| | Heat retention time in temperature range of Ac3 or more (s) | 114 |
| | Average cooling rate in temperature range of 620 °C to 740 °C (°C/s) | 3.5 |
| | Cooling stop temperature (°C) | 681 |
| | Heat retention time in temperature range of 620 °C to 740 °C (s) | 33 |
| | Average cooling rate in temperature range of 400 °C or less (°C/s) | 18 |
| | Cooling stop temperature (°C) | 304 |
| | Heat retention time in temperature range of 150 °C or more and 400 °C or less (s) | 501 |

As Comparative Example 2, a search result in the case where the desired property values of the properties of steel material were set as shown in Table 8 and read as an index is shown in Tables 9 and 10. In this example, instead of searching for a chemical composition of elements in steel and a production condition in a plurality of steps for achieving the desired property values, two candidates for the chemical composition and the production condition, which satisfy any of the tensile strength and the elongation, were presented. A steel material can be designed using such candidates as reference values.

[Table 8]

| Property | Desired property value |
|---|---|
| Tensile strength (MPa) | 1440 |
| Elongation (%) | 13.0 |

[Table 9]

| (Candidate 1 - tens sile strength: 1440 MPa) | | |
|---|---|---|
| Production step | Item | Lower limit |
| Converter and secondary refining | C (%) | 0.177 |
| | Si (%) | 0.63 |
| | Mn (%) | 2.62 |
| | P (%) | 0.015 |
| | S (%) | 0.0009 |
| | Al (%) | 0.035 |
| | N (%) | 0.0029 |
| | Cr (%) | 0 |
| | V (%) | 0 |
| | Sb (%) | 0.008 |
| | Mo (%) | 0 |
| | Cu (%) | 0 |
| | Ni (%) | 0 |
| | Ti (%) | 0.02 |
| | Ni (%) | 0.028 |
| | B (%) | 0.0011 |
| | Ca (%) | 10.0008 |
| Heating furnace and hot rolling | Heating temperature (°C) | 1240 |
| | Finish rolling temperature (°C) | 880 |
| | Coiling temperature (°C) | 560 |
| Annealing | Thickness (mm) | 1.4 |
| | Average heating rate in temperature range of 570 °C or more (°C/s) | 12 |
| | Heating temperature (°C) | 590 |
| | Soaking temperature (°C) | 860 |
| | Heat retention time in temperature range of Ac3 or more (s) | 180 |
| | Average cooling rate in temperature range of 620 °C to 740 °C (°C/s) | 5.3 |
| | Cooling stop temperature (°C) | 630 |
| | Heat retention time in temperature range of 620 °C to 740 °C (s) | 46 |
| | Average cooling rate in temperature range of 400 °C or less (°C/s) | 7 |
| | Cooling stop temperature (°C) | 360 |
| | Heat retention time in temperature range of 150 °C or more and 400 °C or less (s) | 720 |

[Table 10]

| (Candidate 2 - elongation: 13 %) | | |
|---|---|---|
| Production step | Item | Lower limit |
| Converter and secondary refining | C (%) | 0.052 |
| | Si (%) | 0.65 |
| | Mn (%) | 2.59 |
| | P (%) | 0.009 |
| | S (%) | 0.0015 |
| | Al (%) | 0.04 |
| | N (%) | 0.0033 |
| | Cr (%) | 0 |
| | V (%) | 0 |
| | Sb (%) | 0.009 |
| | Mo (%) | 0 |
| | Cu (%) | 0 |
| | Ni (%) | 0 |
| | Ti (%) | 0.022 |
| | Ni(%) | 0.029 |
| | B (%) | 0.0012 |
| | Ca (%) | 0.0006 |
| Heating furnace and hot rolling | Heating temperature (°C) | 1240 |
| | Finish rolling temperature (°C) | 880 |
| | Coiling temperature (°C) | 560 |
| Annealing | Thickness (mm) | 1.4 |
| | Average heating rate in temperature range of 570 °C or more (°C/s) | 9 |
| | Heating temperature (°C) | 600 |
| | Soaking temperature (°C) | 860 |
| | Heat retention time in temperature range of Ac3 or more (s) | 60 |
| | Average cooling rate in temperature range of 620 °C to 740 °C (°C/s) | 7.7 |
| | Cooling stop temperature (°C) | 730 |
| | Heat retention time in temperature range of 620 °C to 740 °C (s) | 12 |
| | Average cooling rate in temperature range of 400 °C or less (°C/s) | 43 |
| | Cooling stop temperature (°C) | 210 |
| | Heat retention time in temperature range of 150 °C or more and 400 °C or less (s) | 260 |

Although the embodiments according to the present disclosure have been described above by way of the drawings and examples, various changes and modifications may be easily made by those of ordinary skill in the art based on the present disclosure. Such various changes and modifications are therefore included in the scope of the present disclosure. For example, the functions included in the means, steps, etc. may be rearranged without logical inconsistency, and a plurality of means, steps, etc. may be combined into one means, step, etc. and a means, step, etc. may be divided into a plurality of means, steps, etc.

For example, the presently disclosed techniques can also be implemented as a program describing processes for realizing the functions of the design aid device 1 described above or a storage medium storing such a program, which are also included in the scope of the present disclosure.

For example, although the foregoing embodiments describe an example in which the design aid device 1 includes the data aggregator 11 and the model generator 12, they may be implemented by another information processing device. In this case, the other information processing device aggregates track record data necessary to generate a mathematical model, and generates the mathematical model. The other information processing device transmits the generated mathematical model to the design aid device 1. The other information processing device may include not only the data aggregator 11 and the model generator 12 but also the database generator 13. In this case, the other information processing device may generate a database and transmit the database to the design aid device 1.

REFERENCE SIGNS LIST

1     design aid device
11    data aggregator
12    model generator
13    database generator
14    search unit
15    presentation unit

**Claims**

1.  A design aid method of aiding in metallic material design by a computer, the design aid method comprising:

    inputting a desired property value to a database and searching for a chemical composition of elements in metal and a production condition, the database being generated using at least one mathematical model in which input information including a chemical composition of elements in metal and a production condition and output information including a property value of the metallic material are associated with each other, and storing, in association with input data of each mesh obtained by partitioning an input range corresponding to the input information into a plurality of intervals, output data of the mathematical model corresponding to the input data; and
    presenting the chemical composition of elements in metal and the production condition corresponding to the desired property value that are obtained in the searching.

2.  The design aid method according to claim 1, wherein the input information includes an index indicating metallic microstructure state.

3.  The design aid method according to claim 1 or 2, wherein the database is generated using a plurality of mathematical models, and
    the plurality of mathematical models are generated for respective types of properties of the metallic material.

4.  A design aid method of aiding in metallic material design by a computer, the design aid method comprising:

    inputting a desired property value to a database and searching the database for an index indicating metallic microstructure state corresponding to the desired property value and a chemical composition of elements in metal and a production condition corresponding to the index indicating metallic microstructure state, the database being generated using at least one first mathematical model in which input information including a chemical composition of elements in metal and a production condition and intermediate output information including an index indicating metallic microstructure state are associated with each other and at least one second mathematical model in which the intermediate output information and output information including a property value of a metallic material are associated with each other, and storing, in association with input data of each mesh obtained by partitioning an input range corresponding to the input information into a plurality of intervals, intermediate output data of the first mathematical model and output data of the second mathematical model corresponding to the input data; and
    presenting the chemical composition of elements in metal and the production condition corresponding to the desired property value.

5. The design aid method according to any one of claims 1 to 4, wherein an interval width of an interval of each mesh varies depending on the input information.

6. The design aid method according to any one of claims 1 to 4, wherein an interval width of an interval of each mesh is determined so that an amount of change in output will be constant.

7. The design aid method according to any one of claims 1 to 6, wherein a range of the input information is limited to a predetermined range based on a predetermined condition.

8. A design aid device that aids in metallic material design, the design aid device comprising:

a search unit configured to input a desired property value to a database and search for a chemical composition of elements in metal and a production condition, the database being generated using at least one mathematical model in which input information including a chemical composition of elements in metal and a production condition and output information including a property value of a metallic material are associated with each other, and storing, in association with input data of each mesh obtained by partitioning an input range corresponding to the input information into a plurality of intervals, output data of the mathematical model corresponding to the input data; and
a presentation unit configured to present the chemical composition of elements in metal and the production condition corresponding to the desired property value that are obtained by the search unit.

# FIG. 1

```
                                              ⌇1
  ┌──────────────────────────────────────────┐
  │                                    ⌇11     │
  │   ┌──────────────────────────────┐        │
  │   │      Data aggregator         │        │
  │   └──────────────────────────────┘        │
  │                                    ⌇12     │
  │   ┌──────────────────────────────┐        │
  │   │      Model generator         │        │
  │   └──────────────────────────────┘        │
  │                                    ⌇13     │
  │   ┌──────────────────────────────┐        │
  │   │     Database generator       │        │
  │   └──────────────────────────────┘        │
  │                                    ⌇14     │
  │   ┌──────────────────────────────┐        │
  │   │        Search unit           │        │
  │   └──────────────────────────────┘        │
  │                                    ⌇15     │
  │   ┌──────────────────────────────┐        │
  │   │     Presentation unit        │        │
  │   └──────────────────────────────┘        │
  │                                            │
  └──────────────────────────────────────────┘
```

# FIG. 2

Components in molten steel

Blast furnace

Converter

Continuous casting

Iron ore

Coke

Limestone

Secondary refining

Heating furnace

Hot rolling

Rapid cooling

Coiling temperature

Heating temperature

Rolling reduction

Finish temperature

Cooling rate

Pickling line

Cold rolling

Annealing

Rolling reduction

Heating temperature
Heat retention time
Cooling rate

Cold-rolled coil

EP 3 913 635 A1

# FIG. 3

Input 3
(Chemical composition or production condition)

Input 2
(Chemical composition or production condition)

Input 1
(Chemical composition or production condition)

Mathematical model
generation

Output
(Property value)

EP 3 913 635 A1

# FIG. 4

# FIG. 5

y2 — Given index of property

Search

Database

| Output (Property) | Input 1 (Chemical composition 1) | Input 2 (Chemical composition 2) | ... | Input m (Production condition 1) | ... |
|---|---|---|---|---|---|
| y1 | x11 | x21 | ... | xm1 | ... |
| y2 | x12 | x22 | ... | xm2 | ... | → Extraction |
| y3 | x13 | x23 | ... | xm3 | ... |
| ⋮ | ⋮ | ⋮ | ⋮ | ⋮ | ⋮ |

x12, x22, ..., xm2, ...

# FIG. 6

```
        ┌─────────┐
        │  Start  │
        └─────────┘
             │
             ▼
┌─────────────────────────────┐
│       Aggregate data        │ ∼ S10
└─────────────────────────────┘
             │
             ▼
┌─────────────────────────────┐
│  Generate mathematical model│ ∼ S20
└─────────────────────────────┘
             │
             ▼
┌─────────────────────────────┐
│      Generate database      │ ∼ S30
└─────────────────────────────┘
             │
             ▼
┌─────────────────────────────┐
│ Search based on desired property value │ ∼ S40
└─────────────────────────────┘
             │
             ▼
┌─────────────────────────────┐
│     Present search result   │ ∼ S50
└─────────────────────────────┘
             │
             ▼
        ┌─────────┐
        │   End   │
        └─────────┘
```

# FIG. 7

Input 3
(Chemical composition, production condition,
or index indicating microstructure)

Mathematical model
generation

Output
(Property value)

Input 2
(Chemical composition, production condition,
or index indicating microstructure)

Input 1
(Chemical composition, production condition,
or index indicating microstructure)

# FIG. 8

| y12, y22, ⋯ | Given index of plurality of properties |

Search

| Output 1 (Property 1) | Output 2 (Property 2) | ⋯ | Input 1 (Chemical composition 1) | Input 2 (Chemical composition 2) | ⋯ | Input m (Production condition 1) | ⋯ |
|---|---|---|---|---|---|---|---|
| y11 | y21 | ⋯ | x11 | x21 | ⋯ | xm1 | ⋯ |
| y12 | y22 | ⋯ | x12 | x22 | ⋯ | xm2 | ⋯ |
| y13 | y23 | ⋯ | x13 | x23 | ⋯ | xm3 | ⋯ |
| ⋮ | ⋮ | ⋮ | ⋮ | ⋮ | ⋮ | ⋮ | ⋮ |

Database

Extraction

| x12, x22, ⋯, xm2, ⋯ |

# FIG. 9

EP 3 913 635 A1

| Output 1 (Property 1) | Output 2 (Property 2) | ... | Input 1 (Chemical composition 1) | Input 2 (Chemical composition 2) | ... | Input m (Production condition 1) | ... | |
|---|---|---|---|---|---|---|---|---|
| y11 | y21 | ... | x11 | x21 | ... | xm1 | ... | |
| y12 | y22 | ... | x12 | x22 | ... | xm2 | ... | ➤ Extraction 1 |
| y13 | y23 | ... | x13 | x23 | ... | xm3 | ... | |
| ⋮ | ⋮ | ... | ⋮ | ⋮ | ... | ⋮ | ... | |
| y1n | y2n | ... | x1n | x2n | ... | xmn | ... | ➤ Extraction 2 |
| ⋮ | ⋮ | ⋮ | ⋮ | ⋮ | ... | ⋮ | ⋮ | |

y12, y22, ⋯  Given index of plurality of properties

Search

Database

x12, x22, ⋯, xm2, ⋯

x1n, x2n, ⋯, xmn, ⋯

# FIG. 10

Input 3
(Chemical composition or production condition)

Input 2
(Chemical composition or production condition)

Input 1
(Chemical composition or production condition)

First mathematical model generation

Output
(Index indicating microstructure)

Input
(Index indicating microstructure)

Second mathematical model generation

Output
(Property value)

# FIG. 11

Partition mesh of input

Input 3

Whole range of expected input

Change of granularity of partition mesh

Mathematical model

Output

Input 2

Input 1

Database generation

EP 3 913 635 A1

FIG. 12

FIG. 13

EP 3 913 635 A1

**EP 3 913 635 A1**

<table>
<tr><td colspan="3"><strong>INTERNATIONAL SEARCH REPORT</strong></td><td>International application No.<br>PCT/JP2019/006147</td></tr>
</table>

**A. CLASSIFICATION OF SUBJECT MATTER**
Int.Cl. G06F17/50(2006.01)i

According to International Patent Classification (IPC) or to both national classification and IPC

**B. FIELDS SEARCHED**

Minimum documentation searched (classification system followed by classification symbols)
Int.Cl. G06F17/50, G06F16/00-16/958, G06Q50/04

Documentation searched other than minimum documentation to the extent that such documents are included in the fields searched
| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2007-047872 A (MITSUBISHI HEAVY INDUSTRIES, LTD.) 22 February 2007, paragraphs [0029]-[0035] (Family: none) | 1-8 |
| A | JP 2003-328030 A (JFE STEEL CORPORATION) 19 November 2003, claims (Family: none) | 1-8 |
| A | JP 8-255190 A (SEKISUI CHEMICAL CO., LTD.) 01 October 1996, abstract (Family: none) | 1-8 |

☐ Further documents are listed in the continuation of Box C.     ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08 May 2019 (08.05.2019) | 21 May 2019 (21.05.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

32

**REFERENCES CITED IN THE DESCRIPTION**

**Patent documents cited in the description**

- JP 2019006145 A
- JP 4393586 B
- JP 5605090 B